# EUROPÄISCHE PATENTSCHRIFT

(11) **EP 2 537 829 B1**
(45) Veröffentlichungstag und Bekanntmachung des Hinweises auf die Patenterteilung: **02.03.2016**
(21) Anmeldenummer: 12174668.9
(22) Anmeldetag: 29.10.2003
(51) Int. Cl.: C07C 303/34, C07C 307/06

(54) **Aminobenzoylsulfonsäureamide und deren Herstellung**
Aminobenzoylsulfonamides and their preparation
Aminobenzoyl-sulfonamides et leur preparation

(30) Priorität: 30.10.2002 DE 10250614
(43) Veröffentlichungstag der Anmeldung: 26.12.2012
(62) Teilanmeldung aus: 03779818.8
(73) Patentinhaber: BASF SE, 67056 Ludwigshafen (DE); Hamprecht, Gerhard, 69469 Weinheim (DE); Puhl, Michael, 69493 Hirschberg (DE); Reinhard, Robert, 67117 Limburgerhof (DE); Seitz, Werner, 68723 Plankstadt (DE)
(72) Erfinder: Hamprecht, Gerhard, 69469 Weinheim (DE); Puhl, Michael, 69493 Hirschberg (DE); Wolf, Bernd, 67136 Fußgönheim (DE); Götz, Norbert, 67547 Worms (DE); Keil, Michael, 67251 Freinsheim (DE); Reinhard, Robert, 67117 Limburgerhof (DE); Sagasser, Ingo, 68623 Lampertheim (DE); Seitz, Werner, 68723 Plankstadt (DE)

(56) Entgegenhaltungen:
- WO-A-01/83459

## Beschreibung

Die Erfindung betrifft ein Verfahren zur Herstellung von Aminobenzoylsulfonsäureamide der Formel IIA.1-a 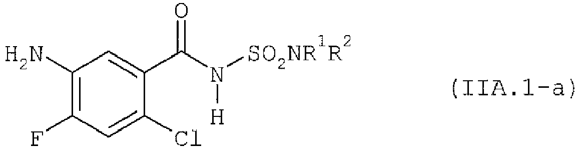
worin R¹ für CH(CH₃)₂; und
R² für CH₃ steht,
dadurch gekennzeichnet, dass Nitrobenzoylsulfamidsäureamide der Formel VA.1-a 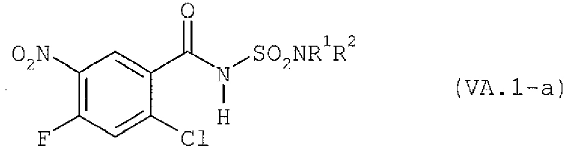 wobei R¹ und R² die zuvor genannten Bedeutungen aufweisen,
mit Wasserstoff in Gegenwart katalytischer Mengen an Übergangsmetallen oder Übergangsmetallverbindungen reduziert werden; sowie die entsprechenden Nitro- und Aminoverbindungen.

WO 01/83459 lehrt, daß im Falle der Herstellung von den erfindungsgemäßen Aminoverbindungen ähnlichen Anilinen durch Reduktion der entsprechenden Nitroverbindungen, die Reduktion vor Einführung der Seitenkette in meta-Position erfolgen sollte.

Überraschender Weise wurde nun gefunden, daß sich die erfindungsgemäßen Aminobenzoylsulfonsäureamide durch Reduktion der entsprechenden Nitrobenzoylsulfamidsäureamide direkt herstellen lassen.

Aminobenzoylsulfonsäureamide sind potentielle Vorstufen für die Herstellung von Iso(thio)cyanatobenzoylsulfamidsäureamiden.

Iso(thio)cyanatobenzoylsulfamidsäureamide sind potentielle Vorstufen für die Herstellung von Pflanzenschutzmitteln mit Triazol-3,5-dion-4-ylgruppe, Pyrimidin-2,6-dion-1-ylgruppe oder 1,3, 5-Triazin-2 , 4, 6-trion-1-ylgruppe oder deren S-Analoga wie sie z.

B. in der WO 01/83459 beschrieben sind.

Grundsätzlich kann man Phenyliso(thio)cyanate durch Umsetzung von primären aromatischen Aminen mit Phosgen beziehungsweise mit Thiophosgen herstellen (siehe z. B. Houben-Weyl, Methoden der organischen Chemie, 4. Auflage, Bd. IX, S. 869, 875-877 und Bd. VIII, S. 120-124). Weitere allgemeine Verfahren sind beispielsweise aus EP 70389, EP 75267 und EP 409 025 bekannt.

Der von einem primären oder sekundären Amin abgeleitete Rest A steht für eine Gruppe der Formel NR¹R²,
worin die Variablen R¹ und R² die folgenden Bedeutungen aufweisen:
- R¹: CH(CH₃)₂; und
- R²: CH₃.

Die Gruppe Ar steht für eine Gruppe der allgemeinen Formel Ar-1 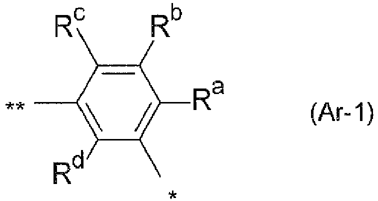 worin
- R^{a}: Chlor;
- R^{b}: Wasserstoff;
- R^{c}: Fluor;
- R^{d}: Wasserstoff.;
- *: die Verknüpfung von Ar mit der C(O)-Gruppe kennzeichnet und
- **: die Verknüpfung von Ar mit dem Stickstoffatom der Amino- bzw. Nitro-Gruppe kennzeichnet.

Die Verbindungen der Formel II können in Analogie zu bekannten Verfahren zur Herstellung von Anilinen gewonnen werden. Die Anilinverbindungen der Formel II kann man beispielsweise gemäß Schema 1 herstellen, indem man zunächst eine Aroylverbindung der Formel III mit einem Sulfamidsäureamid IV im Sinne einer Kondensationsreaktion zu einem N-Aroylsulfamidsäureamid der allgemeinen Formel V umsetzt und anschließend das erhaltene N-Aroylsulfamidsäureamid V zur Verbindung II reduziert.

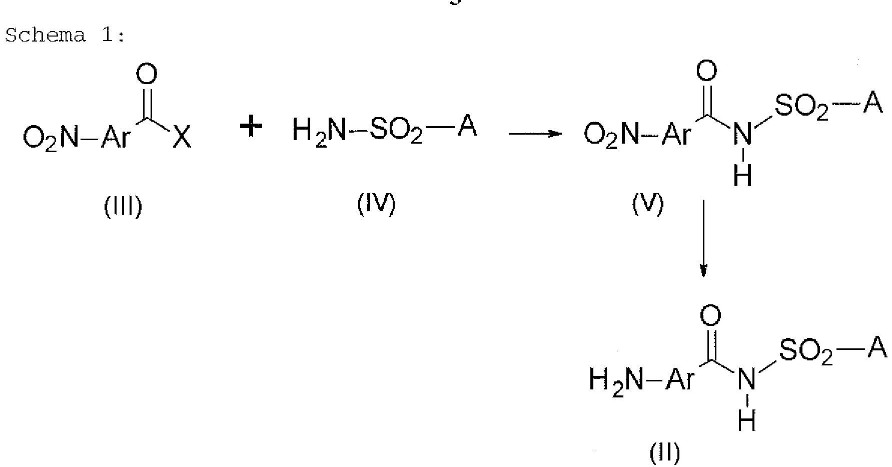

In Schema 1 haben die Variablen A und Ar die vorgenannten Bedeutungen, insbesondere die als bevorzugt angegebenen Bedeutungen. X steht für Halogen, vorzugsweise Chlor, Hydroxy oder eine C₁-C₄-Alkoxygruppe. Die Kondensation von Aroylverbindungen der Formel III mit Sulfamidsäureamiden der Formel IV zu den entsprechenden Benzoylsulfamiden der Formel V erfolgt in Anlehnung an bekannte Verfahren, beispielsweise wie in der WO 01/83459, S. 31-35, in der unveröffentlichten deutschen Patentanmeldung DE 102 21 910.0 beschrieben, auf deren Offenbarung hiermit Bezug genommen wird.

Im Folgenden wird der erste Reaktionsschritt näher erläutert:
Sofern X in Formel III für Hydroxy steht, aktiviert man vorzugsweise zunächst die Carbonsäure III, indem man sie mit einem Kupplungsmittel umsetzt. Anschließend setzt man die aktivierte Carbonsäure III in der Regel ohne vorherige Isolierung mit dem Sulfamidsäureamid IV um. Als Kupplungsmittel kommen beispielsweise N,N'-Carbonyldiimidazol oder Carbodiimide wie Dicyclohexylcarbodiimid in Betracht. Diese werden in der Regel wenigstens in äquimolarer Menge und bis zu einem vierfachen Überschuss, bezogen auf die Carbonsäure III, eingesetzt. Gegebenenfalls erwärmt man das erhaltene Reaktionsgemisch aus Carbonsäure III und Kupplungsmittel und lässt dann auf Raumtemperatur abkühlen. Üblicherweise führt man die Umsetzung in einem Lösungsmittel durch. Als Lösungsmittel kommen z. B. chlorierte Kohlenwasserstoffe wie Methylenchlorid, 1,2-Dichlorethan, Ether z. B. Dialkylether wie Diethylether, Methyl-tert.-butylether oder cyclische Ether wie Tetrahydrofuran oder Dioxan, Carbonsäureamide wie Dimethylformamid, N-Methyllactame wie N-Methylpyrrolidon, Nitrile wie Acetonitril, aromatische Kohlenwasserstoffe wie Toluol, aromatische Amine wie Pyridin oder Gemische hiervon in Betracht. Anschließend versetzt man mit dem Sulfamidsäureamid IV. In der Regel löst man das Sulfamid IV in dem Lösungsmittel, das auch zur Aktivierung der Carbonsäure verwendet wurde.

Sofern X in Formel III für C₁-C₄-Alkoxy steht, kann man zunächst die Ester nach bekannten Verfahren durch Hydrolyse im sauren Milieu unter Verwendung von starken Mineralsäuren wie konzentrierte Salzsäure oder Schwefelsäure oder organischen Säuren wie Eisessig oder Gemischen davon in die entsprechenden Carbonsäuren III überführen. Alternativ lassen sich Ester auch im alkalischen Milieu unter Verwendung von Basen wie Alkalihydroxid, beispielsweise Natriumhydroxid oder Kaliumhydroxid in Gegenwart von Wasser hydrolysieren.

Anschließend kann man die Carbonsäuren III (X = OH) in der oben beschriebenen Weise umsetzen oder mit einem Chlorierungsagens wie Thionylchlorid oder Phosgen zunächst in die Säurechloride (X = Cl) überführen und diese in der nachfolgend beschriebenen Weise mit IV umsetzen. Die Darstellung der Säurechloride erfolgt in Anlehnung an bekannte Verfahren, beispielsweise wie in der EP 1 176 133 und WO 01/087872 beschrieben.

Man kann jedoch auch direkt den Carbonsäureester der Formel III, worin X für C₁-C₄-Alkoxy steht, mit einem Sulfamidsäureamid IV oder dessen Metallsalz im Sinne einer Amidierungsreaktion unter Abspaltung des Esterrestes umsetzen. Die Umsetzung wird man in Anlehnung an die in Houben-Weyl, 4. Auflage, Bd. VIII, S. 658 - 659 beschriebene Arbeitsweise durchführen.

Sofern X in Formel III für Halogen steht, wird man in der Regel so vorgehen, dass man die Aroylverbindung III, vorzugsweise verdünnt in einem inerten Lösungsmittel, zu dem Sulfamidsäureamid der Formel IV, vorzugsweise ebenfalls verdünnt in einem inerten Lösungsmittel, gibt. Selbstverständlich kann man auch die Aroylverbindung III vorlegen und hierzu das Sulfamidsäureamid IV geben.

Die molaren Verhältnisse, in denen die Ausgangsverbindungen III und IV miteinander umgesetzt werden, betragen im Allgemeinen 0,9 bis 1,2, vorzugsweise 0,95 bis 1,1, besonders bevorzugt 0,98 bis 1,04 für das Verhältnis von Aroylverbindung III zu Sulfamidsäureamid IV.

Üblicherweise führt man die Umsetzung bei Temperaturen im Bereich von -30 bis 100 °C, vorzugsweise -10 bis 80 °C, besonders bevorzugt 0 bis 60 °C durch.

Vorteilhaft arbeitet man im ersten Reaktionsschritt unter neutralen Bedingungen. Sofern bei der Reaktion ein saures Reaktionsprodukt, z. B. Halogenwasserstoff (wenn X in Formel III für Halogen steht) entsteht, so entfernt man diesen durch Zugabe einer basischen Verbindung. Zu den geeigneten basischen Verbindungen zählen anorganische und organische Basen. Geeignete anorganische basische Verbindungen sind z. B. Alkali- oder Erdalkalihydroxide bzw. -hydrogencarbonate oder -carbonate. Man kann die Reaktion jedoch auch in Gegenwart einer organischen Base, z. B. Triethylamin, Tri-n-propylamin, N-Ethyldiisopropylamin, Pyridin, α,-, β-, γ-Picolin, 2,4-, 2,6-Lutidin, N-Methylpyrrolidin, Dimethylanilin, N,N-Dimethylcyclohexylamin, Chinolin oder Acridin durchführen. In der Regel setzt man die Base im Überschuss, bezogen auf die Verbindung III, ein. Die molare Menge an Base beträgt 1,0 bis 2 Mol, vorzugsweise 1,02 bis 1,3 Mol Base (berechnet als Basenäquivalent) pro Mol der Verbindung III. Gegebenenfalls enthält das Reaktionsgemisch Pyridin oder eine Pyridinverbindung, beispielsweise ein 4-Dialkylaminopyridin wie 4-Dimethylaminopyridin als Katalysator. Der Zusatz an Katalysator beträgt etwa 0,1 - 10 %, bezogen auf die Verbindung III.

Die Umsetzung der Aroylverbindungen III mit den Verbindungen der Formel IV wird vorteilhaft in Gegenwart eines Lösungsmittels durchgeführt. Als Lösungsmittel verwendet man für diese Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie N,N-Dimethylformamid, N-Methylpyrrolidon, Nitrokohlenwasserstoffe wie Nitrobenzol, Tetraalkylharnstoffe wie Tetraethylharnstoff, Tetrabutylharnstoff, Dimethylethylenharnstoff, Dimethylpropylenharnstoff, Sulfoxide wie Dimethylsulfoxid, Sulfone wie Dimethylsulfon, Diethylsulfon, Tetramethylensulfon, Nitrile wie Acetonitril, Propionitril, Butyronitril oder Isobutyronitril; Wasser oder auch Gemische einzelner Lösungsmittel.

Ferner kann man die Reaktion auch in einem wässrigen Zweiphasensystem durchführen, vorzugsweise in Gegenwart von Phasentransferkatalysatoren wie quartären Ammonium- oder Phosphoniumsalzen. Für die Zweiphasen-Reaktion sind die in der EP-A 556737 beschriebenen Reaktionsbedingungen geeignet.

Als Phasentransferkatalysatoren können quartäre Ammonium- oder Phosphoniumsalze verwendet werden. An geeigneten Verbindungen seien folgende genannt: Tetraalkyl- (C₁-C₁₈) -ammoniumchloride, -bromide oder -fluoride, N-Benzyltrialkyl-(C₁-C₁₈)-ammoniumchloride, -bromide oder -fluoride, Tetraalkyl-(C₁-C₁₈)-phosphoniumchloride oder -bromide, Tetraphenylphosponiumchlorid oder -bromid, (Phenyl)ₒ(C₁-C₁₈-alkyl)ₚ-phosponiumchloride oder -bromide, wobei o = 1 bis 3, p = 3 bis 1 und o + p = 4 ist. Besonders bevorzugt sind Tetraethylammoniumchlorid und N-Benzyltriethylammoniumchlorid. Die Menge an Phasentransferkatalysator beträgt im Allgemeinen bis zu 20 Gew.-%, bevorzugt zwischen 1 und 15 Gew.-% und besonders bevorzugt zwischen 2 bis 8 Gew.-%, bezogen auf die Ausgangsverbindung IV.

Vorteilhaft gibt man die Aroylverbindung III innerhalb eines Zeitraums von 0,25 bis 2 Stunden zu einer Mischung des Sulfamidsäureamids IV und gegebenenfalls der Base in einem der vorgenannten Lösungsmittel und rührt zur Vervollständigung der Reaktion noch 0,5 bis 16 Stunden, vorzugsweise 2 bis 8 Stunden nach. Die Reaktionstemperatur liegt in der Regel zwischen 0 °C und 60 °C.

Bei Verwendung eines wässrigen Zweiphasensystems kann man in beliebiger Reihenfolge die Ausgangsstoffe III und IV zu einer Mischung des Phasentransferkatalysators in den beiden Phasen unter Rühren zugeben und dann im genannten Temperaturbereich unter Zugabe von Base die Umsetzung zu Ende bringen.

Die Reaktion kann kontinuierlich oder diskontinuierlich, drucklos oder unter Druck durchgeführt werden.

Zur Aufarbeitung extrahiert man die organische Phase mit verdünnter Mineralsäure wie Salzsäure, trocknet die organische Phase und entfernt das Lösungsmittel im Vakuum. Gegebenenfalls kann man den Rückstand auch durch Verrühren mit einem Lösungsmittel oder Lösungsmittelgemisch, beispielsweise aromatische Kohlenwasserstoffe wie Benzol, Xylol oder Toluol oder aliphatische oder cycloaliphatische Kohlenwasserstoffe wie Petrolether, Pentan, Hexan oder Cyclohexan, Ether wie Diethylether usw. und Gemischen davon, Absaugen und Trocknen noch weiter reinigen.

Im Folgenden wird der 2. Reaktionsschritt, die Reduktion der Nitroverbindung V zu der Verbindung II, näher erläutert.

Die Aufarbeitung zur Gewinnung des Zielproduktes kann nach den hierfür üblichen Verfahren erfolgen. In der Regel wird man zunächst das Lösungsmittel entfernen, beispielsweise durch Destillation. Zur weiteren Reinigung kann man übliche Verfahren wie Kristallisation, Chromatographie, beispielsweise an Kieselgel, Verühren mit einem Lösungsmittel, beispielsweise aromatische Kohlenwasserstoffe wie Benzol, Toluol oder Xylol oder aliphatische Kohlenwasserstoffe wie Petrolether, Hexan, Cyclohexan, Pentan, Carbonsäureester wie Essigsäureethylester usw. und Gemische davon anwenden.

Ein geeignetes Reduktionsmittel für die Umwandlung der Verbindung V in die Verbindung II ist Wasserstoff in Gegenwart katalytischer Mengen an Übergangsmetallen oder Übergangsmetallverbindungen, insbesondere der 8. Nebengruppe. Bevorzugte Übergangsmetalle sind beispielsweise Nickel, Palladium, Platin, Ruthenium oder Rhodium. Die Übergangsmetalle können als solche oder in geträgerter Form eingesetzt werden. Beispiele für Träger sind Aktivkohle, Aluminiumoxid, ZrO₂, TiO₂, SiO₂, Carbonate und dergleichen. Die Übergangsmetalle können auch in Form aktivierter Metalle wie Raney-Nickel eingesetzt werden. Die Übergangsmetalle können auch in Form von Verbindungen eingesetzt werden. Geeignete Übergangsmetallverbindungen sind z. B. Palladiumoxid und Platinoxid. Die Katalysatoren werden im Allgemeinen in einer Menge von 0,05 bis 10,0 Mol-% (gerechnet als Metall), bezogen auf die zu reduzierende Verbindung V, eingesetzt. Man arbeitet entweder lösungsmittelfrei oder in einem inerten Lösungs- oder Verdünnungsmittel. Geeignete Lösungsmittel oder Verdünnungsmittel für die Reduktion sind je nach Löslichkeit des zu hydrierenden Substrates und dem gewählten Reduktionsmittel, z. B. Carbonsäuren wie Essigsäure, oder wässrige Lösungen organischer Säuren wie Essigsäure und Wasser, Carbonsäureester wie Essigsäureethylester, C₁-C₄-Alkohole wie Methanol, Ethanol, n-Propanol, Isopropanol, n-Butanol oder aromatische Kohlenwasserstoffe wie Toluol. Nach Abtrennen des Katalysators kann die Reaktionslösung wie üblich auf das Produkt hin aufgearbeitet werden. Die Hydrierung kann bei Normalwasserstoffdruck oder unter erhöhtem Wasserstoffdruck, beispielsweise bei einem Wasserstoffdruck von 0,01 bis 50 bar, vorzugsweise 0,1 bis 40 bar, durchgeführt werden. Zur katalytischen Hydrierung aromatischer Nitroverbindungen siehe beispielsweise Rylander in "Catalytic Hydrogenation over PlatinumMetalls" , Academic Press, New York, 1967, 168-202; Furst et al. Chem. Rev. 1965, 65, 52; Tepko et al., J. Org. Chem. 1980, 45, 4992.

Bei chlorhaltigen Benzoylsulfamiden hydriert man je nach Empfindlichkeit des Substituenten bei 20 bis 170 °C, zweckmäßig 20 bis 140 °C, vorteilhaft 20 bis 80 °C. Bei reaktiven Halogensubstituenten empfiehlt es sich ferner in neutraler Lösung, eventuell bei nur leicht erhöhtem Druck mit geringen Mengen an Nickel-, Platin- oder auch Rhodiumkatalysatoren zu hydrieren; geeignet sind auch Edelmetallsulfide wie Platinsulfid. Das Verfahren ist im Houben-Weyl "Methoden der organischen Chemie", Bd. IV/1C, S. 520-526 eingehend beschrieben.

Die in Schema 1 eingesetzten Aroylverbindungen III sind nach an sich im Stand der Technik bekannten Verfahren erhältlich oder lassen sich in Anlehnung an bekannte Verfahren herstellen, beispielsweise gemäß US 6,251,829, EP 415 641, EP 908 457, EP 1176133 und WO 01/087872.

Die Sulfamidsäureamide IV sind im Stand der Technik bekannt oder lassen sich nach bekannten Verfahren, z. B nach der deutschen Patentanmeldung DE 102 21 910.0 durch Umsetzung von Ammoniak mit Sulfamidsäurehalogeniden herstellen. Auf die Offenbarung dieser Schrift wird hiermit Bezug genommen.

Vorzugsweise stellt man die Sulfamidsäureamide IV nach dem in der unveröffentlichten deutschen Patentanmeldung DE 102 21 910.0 beschriebenen Verfahren her. Dieses Verfahren umfasst die folgenden Schritte: (i) Umsetzung eines primären oder sekundären Amins mit wenigstens einer äquimolaren Menge SO₃ oder einer SO₃-Quelle in Gegenwart von wenigstens äquimolaren Mengen eines tertiären Amins, jeweils bezogen auf das primäre oder sekundäre Amin, wobei man ein Amidosulfonsäureammoniumsalz erhält; (ii) Umsetzung des Amidosulfonsäureammoniumsalzes mit wenigstens einer stöchiometrischen Menge eines Phosphorhalogenids, wobei man ein Sulfamidsäurehalogenid erhält und (iii) Umsetzung des in Schritt ii) erhaltenen Sulfamindsäurehalogenids mit Ammoniak, wobei man das Sulfamidsäureamid V erhält.

Das erfindungsgemäße Verfahren ist dem in der WO 01/83459 beschriebenen Verfahren überlegen.

Die folgenden Beispiele dienen der Erläuterung der Erfindung

### I Herstellung der Nitrobenzoylsulfamidsäureamide (Vorstufe der allgemeinen Formel VA.1):

### Beispiel 1:

### N-(2-Chlor-4-fluor-5-nitro-benzoyl)-N'-n-propyl-N'-allylsulfamid (VA.1-a.241; nicht erfindungsgemäß)

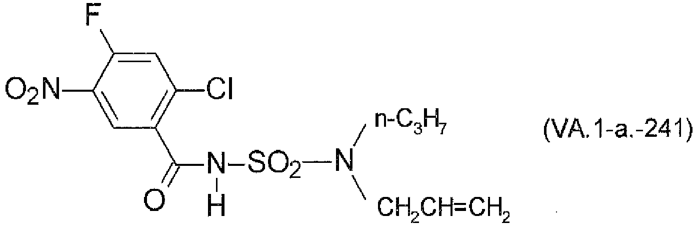

Bei -5 °C bis 0 °C gab man zu einer Mischung von 8,50 g (0,048 mol) N'-Propyl-N'-allylsulfamid, 10,38 g (0,103 mol) Triethylamin und 0,09 g (0,736 mmol) 4-N,N-Dimethylaminopyridin in 90 ml Methylenchlorid unter Rühren innerhalb 30 Minuten 11,62 g (0,0474 mol) 2-Chlor-4-fluor-5-nitrobenzoylchlorid in 50 ml Methylenchlorid. Man spülte mit 10 ml des Lösungsmittels nach. Man rührte zunächst 1 Stunde bei 0 °C und anschließend 2 Stunden bei 22 °C nach. Anschließend gab man 50 ml 1N Salzsäure zu, rührte und trennte die Phasen. Man wusch die organische Phase noch zweimal mit 1N Salzsäure und extrahierte die wässrige Phase mit Methylenchlorid. Nach dem Trocknen der organischen Phase über Magnesiumsulfat filtrierte man und engte die Lösung ein. Den Rückstand verrührtemanmit Diethylether/Pentan, saugte ab und trocknete, wobei man 18,41 g (91,9% der Theorie) der Titelverbindungmit einem Schmelzpunkt (Schmp. ) von 110-112 °C erhielt.

In analoger Weise wurde die in Tabelle 1 angegebene Vorstufe VA.1-a.86 erhalten.

**Tabelle 1:**

| | | | | | |
|---|---|---|---|---|---|
| 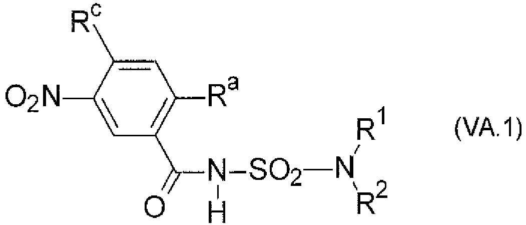 | | | | | |

| Beispiel / Nr. | R^{c} | R^{a} | R¹ | R² | Schmp. [ °C] |
|---|---|---|---|---|---|
| 2 = VA.1-a.86 | F | Cl | CH₃ | i-C₃H₇ | 121 - 122 |

### II Herstellung der Aminobenzoylsulfamidsäureamide der allgemeinen Formel IIA (Vorprodukte IIA.1):

### IIa Reduktion der Nitrogruppe mit Eisenpulver in Essigsäure (nicht erfindungsgemäß)

### Beispiel 3 (nicht erfindungsgemäß):

### N-(5-Amino-2-chlor-4-fluor-benzoyl)-N'-allyl-N'-n-propylsulfamid (IIA.1-a.241)

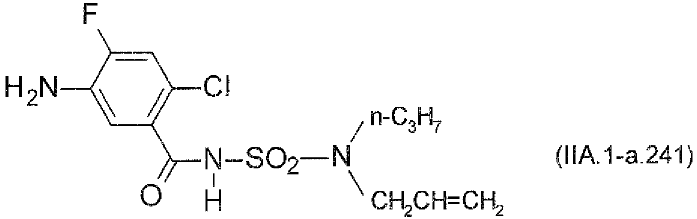

Zu einer Suspension von 7,54 g (135,072 mmol) Eisenpulver in 60 ml Essigsäure gab man unter Rühren innerhalb 25 Minuten eine Lösung von 17,1 g (45,02 mmol) der Verbindung VA.1-a.241 aus Beispiel 1 in einer Mischung von 5 ml Tetrahydrofuran und 40 ml Essigsäure bei 70 bis 75 °C. Man rührte noch 1 Stunde bei 70 bis 75 °C nach, ließ abkühlen und engte im Vakuum ein. Man rührte den Rückstand mit Essigester, filtrierte und wusch den Niederschlag mit Essigester. Man rührte das Filtrat mit Aktivkohle und Magnesiumsulfat, filtrierte, wusch und engte ein. Nach dem Anteigen des Rückstands mit Essigester, Verrühren mit Pentan, Absaugen und Trocknen erhielt man 12,1 g (75,3% der Theorie) der Titelverbindung mit einem Schmelzpunkt von 104 bis 106 °C.

### IIb Katalytische Hydrierung der Nitrogruppe

### Beispiel 4: N-(5-Amino-2-chlor-4-fluor-benzoyl)-N'-methyl-N'-isopropylsulfamid (IIA.1-a.86)

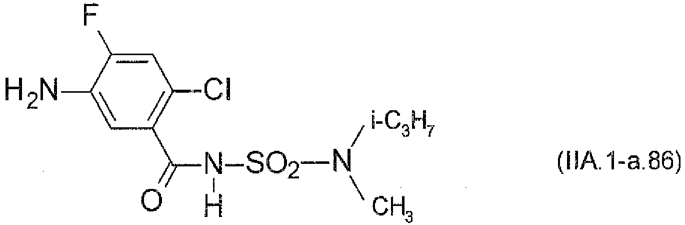

Man legte 112,0 g (0,317 mol) der Verbindung VA.1-a.86 aus Beispiel 7 und 100 g Raney-Nickel in 1200 ml Methanol in einer Hydrierapparatur vor. Unter Rühren spülte man mit 10 l Stickstoff und mit 10 l Wasserstoff. Unter Rühren hydrierte man bei 22 - 23 °C mit 0,1 bar Wasserstoff. Insgesamt wurden 21,3 l Wasserstoff aufgenommen. Nach dem Abblasen des Überdrucks spülte man erneut mit 10 l Stickstoff. Man saugte das Reaktionsgemisch über Kieselgel ab und engte das Filtrat im Vakuum ein. Man erhielt 100, 5 g (97 % der Theorie) der Titelverbindung mit einem Schmelzpunkt von 160 - 162 °C (HPLC-Reinheit: 99,1 %).

## Patentansprüche

1. Verfahren zur Herstellung von Aminobenzoylsulfonsäureamiden der Formel IIA.1-a 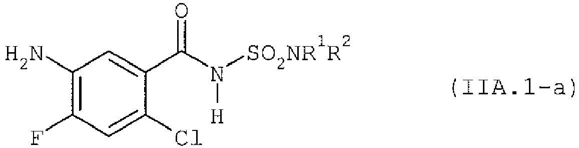
worin R¹ für CH(CH₃)₂; und
R² für CH₃ steht;
**dadurch gekennzeichnet, dass** Nitrobenzoylsulfamidsäureamide der Formel VA.1-a 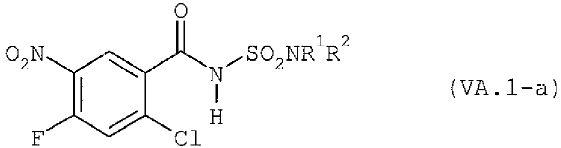 wobei R¹ und R² die zuvor genannten Bedeutungen aufweisen,
mit Wasserstoff in Gegenwart katalytischer Mengen an Übergangsmetallen oder Übergangsmetallverbindungen reduziert werden.

2. Verfahren gemäß Anspruch 1, **dadurch gekennzeichnet, dass** das Verfahren folgende Schritte umfasst:
a) Umsetzen einer Aroylverbindung der Formel IIIA.1-a 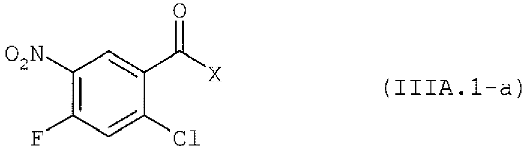 wobei X für Halogen oder C₁-C₄-Alkoxy steht,
mit einem Sulfamidsäureamid der Formel IVA.1-a
H₂N-SO₂-NR¹R² (IVA.1-a)
wobei R¹ für CH(CH₃)₂; und
R² für CH₃ steht; und
b) Reduktion des in Stufe a) erhalten Nitrobenzoylsulfamidsäureamids der Formel VA.1-a 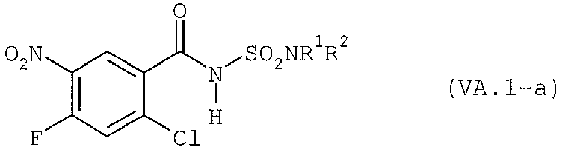 wobei R¹ und R² die oben genannten Bedeutungen haben
mit Wasserstoff in Gegenwart katalytischer Mengen an Übergangsmetallen oder Übergangsmetallverbindungen.

3. Aminobenzoylsulfonsäureamide der Formel IIA.1-a 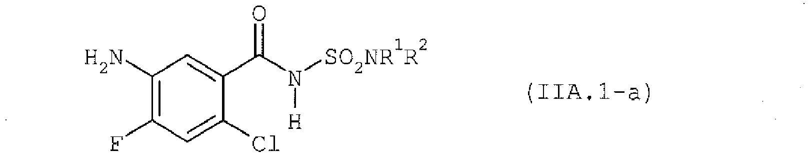
worin R¹ für CH(CH₃)₂; und
R² für CH₃ steht.

4. Nitrobenzoylsulfonsäureamide der Formel VA.1-a 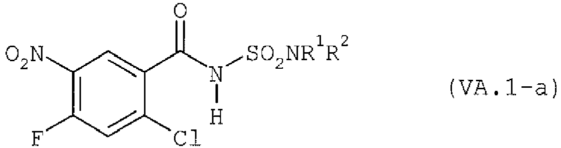
worin R¹ für CH(CH₃)₂; und
R² für CH₃ steht.

## Claims

1. A process for preparing aminobenzoylsulfonamides of the formula IIA.1-a 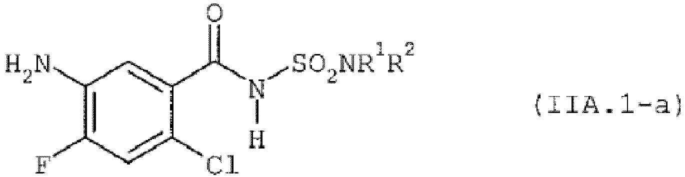
in which R¹ is CH(CH₃)₂ and
R² is CH₃,
which comprises reducing nitrobenzoylsulfonamides of the formula VA.1-a 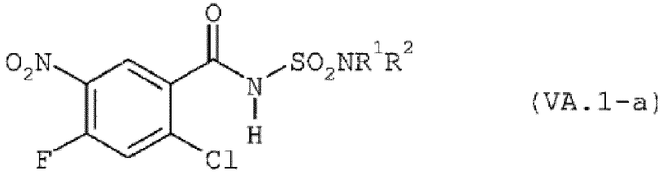 where R¹ and R² have the meanings mentioned above
with hydrogen in the presence of catalytic amounts of transition metals or transition metal compounds.

2. The process as claimed in claim 1, wherein the process comprises the following steps:
a) reaction of an aroyl compound of the formula IIIA.1-a 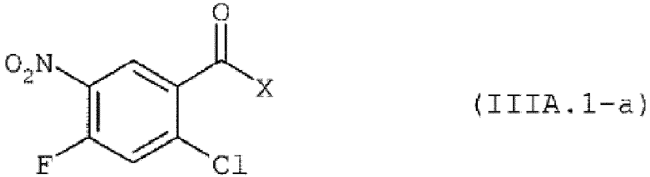 where X is halogen or C₁-C₄ alkoxy
with a sulfonamide of the formula IVA.1-a
H₂N-SO₂-NR¹R² (IVA.1-a)
where R¹ is CH(CH₃)₂ and
R² is CH₃ and
b) reduction of the nitrobenzoylsulfonamide, obtained in step a) of the formula VA.1-a 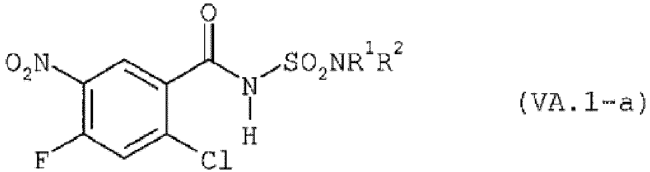 where R¹ and R² have the meanings mentioned above
with hydrogen in the presence of catalytic amounts of transition metals or transition metal compounds.

3. An aminobenzoylsulfonamide of the formula IIA.1-a 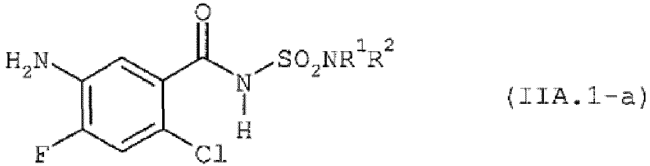
in which R¹ is CH(CH₃) and
R² is CH₃.

4. A nitrobenzoylsulfonamide of the formula VA.1-a 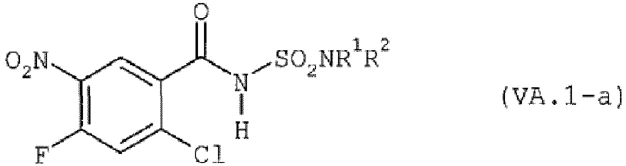
in which R¹ is CH(CH₃)₂ and
R² is CH₃.

## Revendications

1. Procédé de fabrication d'amides de l'acide aminobenzoylsulfonique de formule IIA.1-a 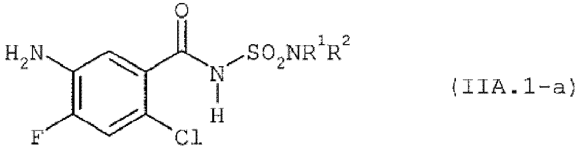 dans laquelle
R¹ représente CH(CH₃)₂ ; et
R² représente CH₃ ;
**caractérisé en ce que** des amides de l'acide nitrobenzoylsulfamidique de formule VA.1-a 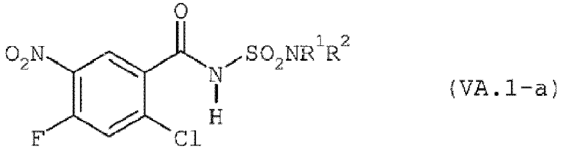 dans laquelle R¹ et R² ont les significations indiquées précédemment,
sont réduits avec de l'hydrogène en présence de quantités catalytiques de métaux de transition ou de composés de métaux de transition.

2. Procédé selon la revendication 1, **caractérisé en ce que** le procédé comprend les étapes suivantes :
a) la mise en réaction d'un composé d'aroyle de formule IIIA.1-a 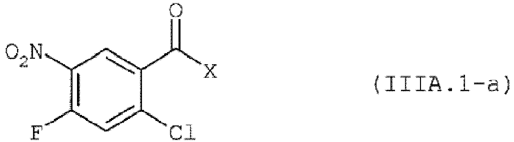 dans laquelle X représente un halogène ou alcoxy en C₁-C₄.
avec un amide de l'acide sulfamidique de formule IVA.1-a
H₂N-SO₂-NR¹R² (IVA.1-a)
dans laquelle
R¹ représente CH(CH₃)₂ ; et
R² représente CH₃ ; et
b) la réduction de l'amide de l'acide nitrobenzoylsulfamidique de formule VA.1-a 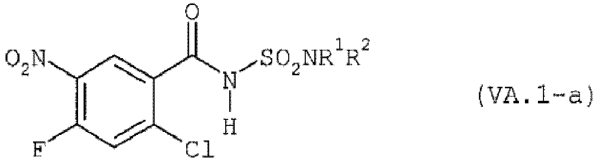 dans laquelle R¹ et R² ont les significations indiquées précédemment,
avec de l'hydrogène en présence de quantités catalytiques de métaux de transition ou de composés de métaux de transition.

3. Amides de l'acide aminobenzoylsulfonique de formule IIA.1-a 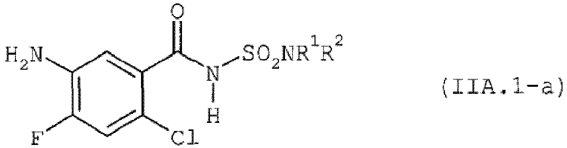 dans laquelle
R¹ représente CH(CH₃)₂ ; et
R² représente CH₃.

4. Amides de l'acide nitrobenzoylsulfonique de formule VA.1-a 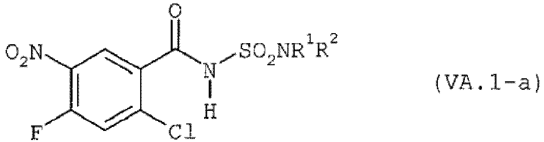 dans laquelle
R¹ représente CH(CH₃)₂ ; et
R² représente CH₃.
